# EUROPEAN PATENT APPLICATION

(11) **EP 0 568 774 A1**
(43) Date of publication of application: **10.11.1993**
(21) Application number: 93102119.0
(22) Date of filing: 11.02.1993
(51) Int. Cl.: A61B 17/115

(54) **Endoscopic anastomosis ring insertion device**

(30) Priority: 08.04.1992 US 865235
(71) Applicant: AMERICAN CYANAMID COMPANY, Wayne, NJ 07470-8426 (US)
(72) Inventor: Allen, William J., Stratford, Connecticut 06496 (US); Jessup, George, Strathfield, NSW 2135 (AU); Howard, John F., Brookfiled, Connecticut 06804 (US); Terk, Harold S., Stamford, Connecticut 06903 (US); Miller, Lester F., Danbury, Connecticut 06811 (US); Ahari, Frederick F., Southport, Connecticut 06490 (US)
(74) Representative: Wächtershäuser, Günter, Prof. Dr.

(57) **Abstract**

An endoscopic insertion device for inserting an anastomosis ring (11) having two unitary members (13) in an anatomic tubular body organ comprises an anastomosis ring adapter (16) for mounting the unitary members in an engaging position, an inserter for inserting the ring adapter in the tubular body organ, and an actuator for actuating the adapter to close the anastomosis ring (11). The anastomosis ring (11) is closed to clamp together open ends of the tubular body organ and then the ring adapter is released from the closed anastomosis ring.

## Description

This invention relates to a surgical device, and more particularly to an anastomosis ring insertion device for use in anastomosing tubular body organs.

The insertion device of the subject invention inserts the anastomosis ring into the human body using a non-invasive surgical procedure. Such a procedure entails inserting the anastomosis ring through a natural body orifice. The non-invasive procedure has several advantages over invasive surgery, which inserts the anastomosis ring through a bodily incision proximate to the severed ends of the body organ, such as quicker healing time and reduced risk of infection for the patient.

The subject invention can be used, for example, to anastomose the esophagus by inserting the anastomosis ring through the mouth. As another example, the insertion device of the subject invention can insert the anastomosis ring through the anal orifice of the patient to anastomose the lower colon, or bowel section, of the intestines.

After a surgical procedure such as cutting and removing a diseased or cancerous portion of a tubular body organ, the severed ends of the body organ must be anastomosed. Several procedures are available for connecting together two sections of hollow tubular body organs such as the intestines. Known procedures include suturing, stapling or clamping the severed ends together. For example, U.S. Patents No. 4,576,167, No. 4,603,693 and No. 4,646,745 are directed to circular surgical staplers for joining hollow body organs.

Another procedure using anastomosis buttons and clamps is disclosed in U.S. Patents No. 3,771,526, No. 4,055,186 and No. 4,154,241. These devices may utilize inserter rods which are forced upwardly into the rectum through the anus to position one half of a clamp device in the lower colon and engage the other half of the clamp device positioned in the upper colon to draw the two halves together.

Still another procedure involves use of an anastomotic device that is the subject of U.S. Patents No. 4,467,804, No. 4,552,148 and No. 4,766,898, which are assigned to the assignee of the present application. The insertion device of the subject invention is used in conjunction with an anastomotic device of the type disclosed in these patents. The anastomotic device receives the open ends of two tubular body organs to be anastomosed over a pair of ring members. The ring members have annular connecting means which mate with each other to clamp the body organs contiguous to each other so they can grow and heal together.

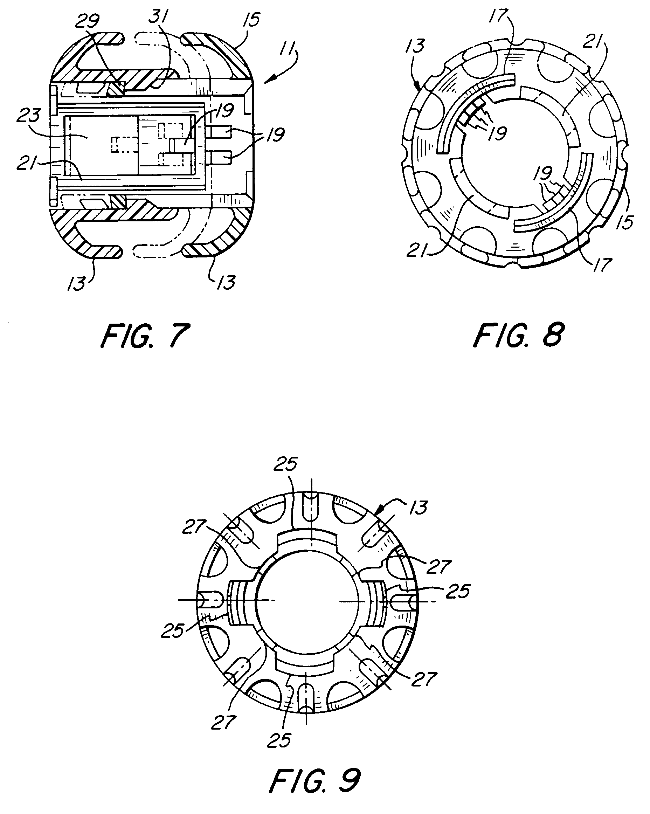

The anastomotic device, which will be referred to hereinafter and in its embodiment for use with the subject invention as an anastomosis ring, is pictured in Figures 7 through 9. A complete anastomoses ring 11 is shown in Figure 7 and is comprised of two identical unitary members 13 of mushroom cap configuration. Figures 8 and 9 show a bottom plan view and a top plan view, respectively, of the unitary member. The bottom plan view in Figure 8 shows a ring member 15 having a pair of diametrically opposed depending legs 17 each supporting a plurality of engaging pawls 19. Alternately positioned between the depending legs and opposite to each other are depending engaging members 21, each of which has a pawl engaging recess 23 to cooperatively receive the pawls when the two unitary members 13 are joined together to form the anastomosis ring. For ease of reference, the ring member will be referred to as the head of the anastomosis ring and the depending legs and engaging members will be referred to as the neck.

Figure 9 shows the top of the unitary member molded to form four notches 25 and four ridges 27 alternately positioned around its inner periphery.

As shown in Figure 7, the pawls 19 are positioned on each depending leg 17 such that the unitary members can mate in an engaged position as shown in solid lines, or in a fully closed position as shown in solid and broken lines. As will be discussed in greater detail below, the unitary members are mounted on the insertion device of the subject invention in the engaged position and then inserted into the body. A profile of the engaging pawl showing engaging edge 29 and slope 31 can also be seen in Figure 7.

The unitary members 13 are formed of a bio-absorbable bio-fragmentable material that permits disintegration of the device in a relatively short period of time after healing of the tubular body organ ends begins. Acceptable materials for forming the anastomosis rings are disclosed in U.S. Patent No. 3,297,033 and are referred to as poly-hydroxyacetic ester and lactide copolymers. Molded surgical articles made from a wide variety of glycolide/lactide copolymers are well known in the art.

U.S. Patent No. 4,667,673, also assigned to the assignee of the present application, discloses an applicator device for mounting and inserting the anastomosis ring in a bowel section of the patient and a method for using the applicator. The device includes a mounting extension for mounting the two halves of the anastomosis ring and an inserter which may be curved. The two halves are spaced apart from each other in the mounted position and then drawn together by manipulation of the inserter. The inserter portion of the applicator passes through the interior of the rectum and out through the exterior of the anus so that the placement of the anastomosis ring can be done without being exposed to the dirty and contaminated end of the bowel.

As a further improvement in an applicator device for inserting an anastomosis ring, the present invention is directed to an endoscopic insertion device and provides advantages for mounting and inserting the anastomosis ring in, for example, the esophagus or the intestines using a non-invasive surgical procedure.

It is a principal object of the present invention to improve upon and enhance an applicator insertion device for surgically inserting an anastomosis ring.

Accordingly, it is an object of the invention to provide an insertion device for surgically inserting an anastomosis ring in a tubular body organ through a natural body orifice.

It is a further object of the invention to provide an insertion device that swiftly and easily mates the unitary members of the anastomosis ring to clamp the open ends of the body organ in a contiguous manner.

It is still a further object of the invention to provide an insertion device that quickly releases a mounting adapter from the anastomosis ring and withdraws the adapter through a natural body orifice.

These and other objects are achieve by the endoscopic insertion device of the present invention, which in a preferred embodiment comprises anastomosis ring adapter means for mounting two unitary members in an engaged position, inserting means for inserting the adapter means in a tubular body organ, and actuating means for actuating the adapter means to close the anastomosis ring and release the adapter means from the anastomosis ring.

In another preferred embodiment, an endoscopic insertion device for inserting an anastomosis ring having two unitary members into an anatomic tubular body organ comprises an anastomosis ring adapter having a cylindrical collar, a sliding cage mounted for axial movement within the collar, and a spool mounted for axial movement within the sliding cage. An endoscope having a biopsy port is connected at its distal end to the collar, and a handle assembly is connected to a proximal end of the endoscope and includes a pivotable trigger. In addition, a control rod is connected at a first end to the trigger and at a second end to the spool.

These and other objects, aspects, features and advantages of the present invention will become apparent from the following detailed description of the preferred embodiment taken in conjunction with the drawings.
Figure 1A is a side view of an endoscope with an attached handle assembly in accordance with the present invention;
Figure 1B is a side view of an adapter with an anastomosis ring mounted ready for use in accordance with the present invention;
Figure 2 is an exploded view, in perspective, of the adapter in accordance with the present invention;
Figure 3A is a cross-sectional side view of the adapter with the anastomosis ring in an "open" position in accordance with the present invention;
Figure 3B is a side view of the handle assembly locked in the "open" position in accordance with the present invention;
Figure 4A is a cross-sectional side view of the adapter with the anastomosis ring in a "closed" position to clamp the open ends of the body organ together in accordance with the present invention;
Figure 4B is a side view of the handle in the "closed" position in accordance with the present invention;
Figure 5A is a cross-sectional view of the adapter in the "release" position in accordance with the present invention;
Figure 5B is a side view of the handle in the "release" position in accordance with the present invention;
Figure 6 is a cross-sectional view of the adapter being withdrawn from the anastomosis ring in accordance with the present invention;
Figure 7 is a cross-sectional side view of the anastomosis ring;
Figure 8 is a bottom plan view of a unitary member of the anastomosis ring; and
Figure 9 is a top plan view of the unitary member of the anastomosis ring.

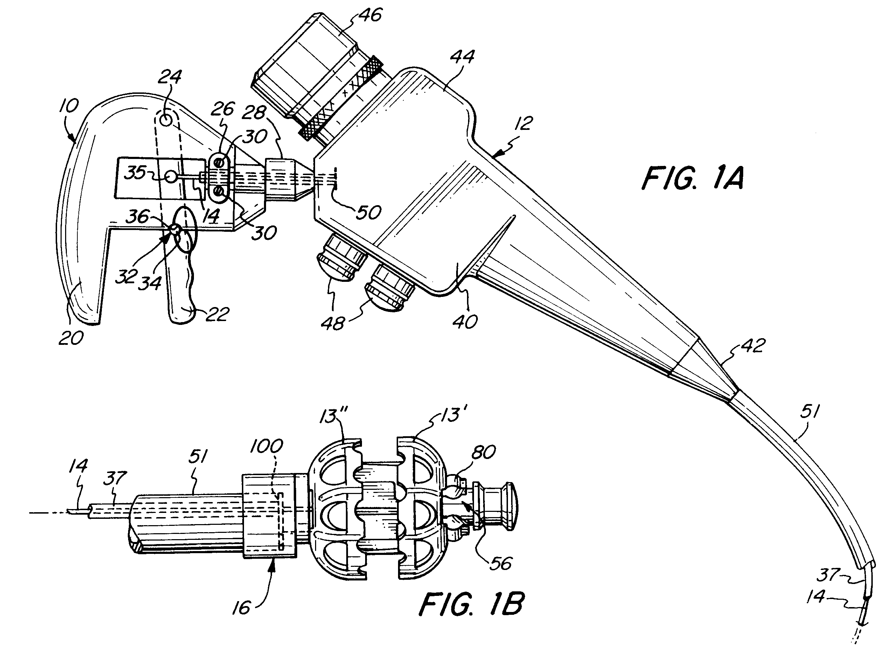

Figures 1A and 1B illustrate the three main components of the endoscopic anastomosis ring insertion device. Figure 1A shows a handle assembly 10 and an endoscope 12. The handle assembly is connected to a proximal end of the endoscope and actuates a control cable 14. The control cable extends through the endoscope and is connected to an anastomosis ring adapter 16. The control cable includes a spherical fastener 35 at each end and has a control cable sheath 37 extending substantially the entire length of the control cable. The ring adapter is shown generally in Figure 1B with an anastomosis ring mounted thereon in the engaged position. The anastomosis ring is formed from the engagement of a distal unitary member 13' and a proximal unitary member 13''.

As used herein, the term "distal" will refer to that part of the device which is furthest away from the surgeon-user, and the term "proximal" refers to that part of the device which is closest to the surgeon-user.

The handle assembly shown in Figure 1A comprises a handle body 20 and a trigger 22 pivotably mounted on a pivot pin 24. The control cable is connected to the trigger and passes through a guide clamp 26 and cable adapter 28 in the handle assembly. The guide clamp provides a passageway 39 for receiving the control cable and is secured to the handle body by, for example, screws 30. The cable adapter connects the handle assembly with the endoscope to form a single unit.

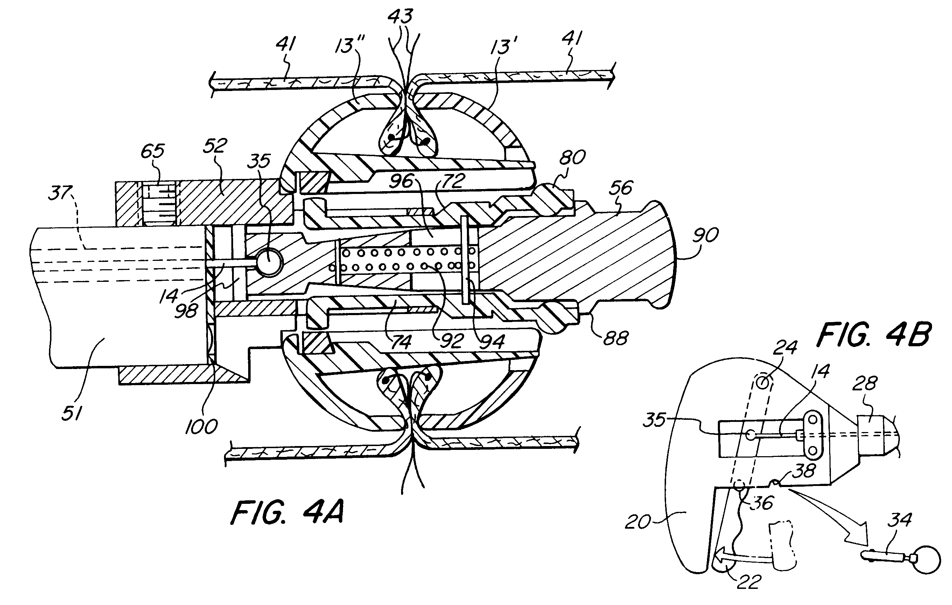

The handle also includes a locking mechanism 32 for locking the trigger. The locking mechanism includes a locking pin 34 that can be inserted through a locking hole 36 in the trigger to engage a locking notch 38 in the handle body and prevent the trigger from pivoting about the pivot pin 24. The locking hole, the locking notch and the locking pin are best seen in Figure 4B.

The endoscope 12 shown in Figure 1A is, per se, known in the art. The endoscope typically includes an elongated body 40 with a distal end 42 and a proximal end 44. At the proximal end is an eye-piece 46 and control knobs 48. Alternatively, the endoscope may be equipped to provide images of the patient electronically. The control knobs are used to control and manipulate, for example, a light cable and an optical cable running through the endoscope. An endoscopic guide 51 extends from the distal end of the elongated body to provide a passageway for the control cable and any other cables in the endoscope. The control cable and sheath enter the endoscope through a biopsy port 50 and extend through the endoscopic guide. As shown in Figure 1B, a large washer 100 fits flush on the distal end of the endoscopic guide to restrain passage of the control cable sheath.

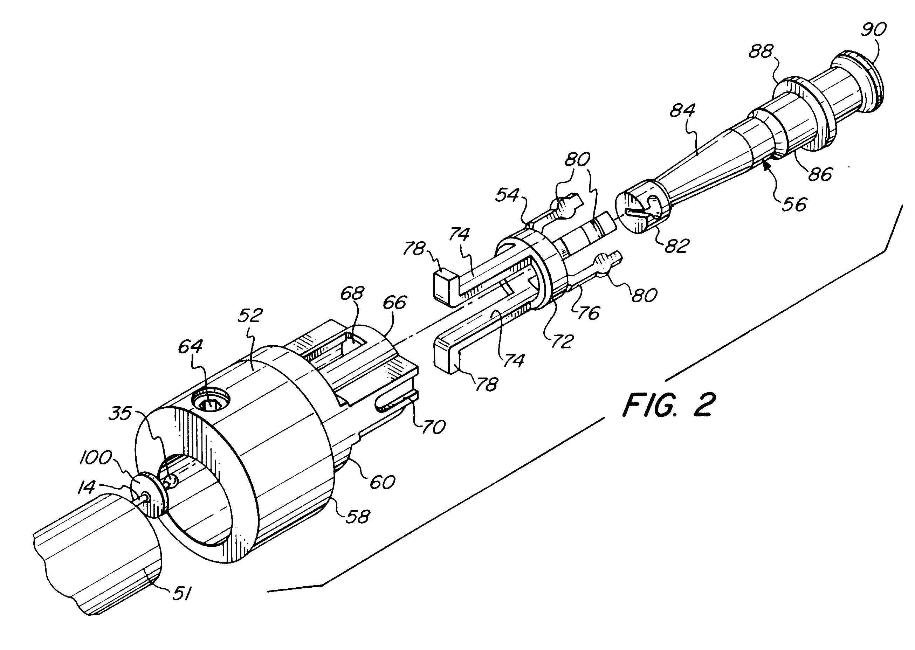

With reference to Figures 2 and 3A, the anastomosis ring adapter 16 comprises generally a cylindrical collar 52, a sliding cage 54 and a spool 56. The collar has a main section 58 and a secondary section 60 of a reduced diameter. The main section is secured to the distal end of the endoscopic guide by a bolt 62, or other fastening means, extending through a transverse opening 64 in the main section.

The secondary section has a hollow sleeve 66 for receiving the cage. The sleeve includes two diametrically opposed elongated slots 68. Two diametrically opposed elongated notches 70 are positioned between the slots.

The sliding cage has an annular rim 72 with two sliding fingers 74 extending in a first axial direction and a plurality, such as four, containing fingers 76 extending in a second axial direction opposite to the first direction. Each sliding finger includes an upturned lip 78 that slides within one of the elongated slots 68 in the hollow sleeve. Each containing finger includes an enlarged contact point 80 for contacting the anastomosis ring and the spool in a manner that will be described below. The contact point shown in the figures is circular in shape.

The spool is formed to have a proximal end 82, a truncated cone section 84 and a distal cylindrical section 86. The cylindrical section has an abutting annular ring 88 formed thereon and a rounded knob-shaped end 90 for easing the insertion device through the tubular body organ. The spool also includes an internal spring 92 for biasing the spool relative to the sliding cage. The spring is connected at its free end to a transverse pin 94 which extends through elongated slots 96 in the spool and is anchored in the annular rim of the cage. The spool is connected at its proximal end to the control rod by the distal spherical fastener 35 and a small washer 98. As shown, for example, in Figure 3A, the small washer is secured flush against the proximal end. The control rod is manipulated by the handle assembly to slide the spool back and forth within the collar and the cage.

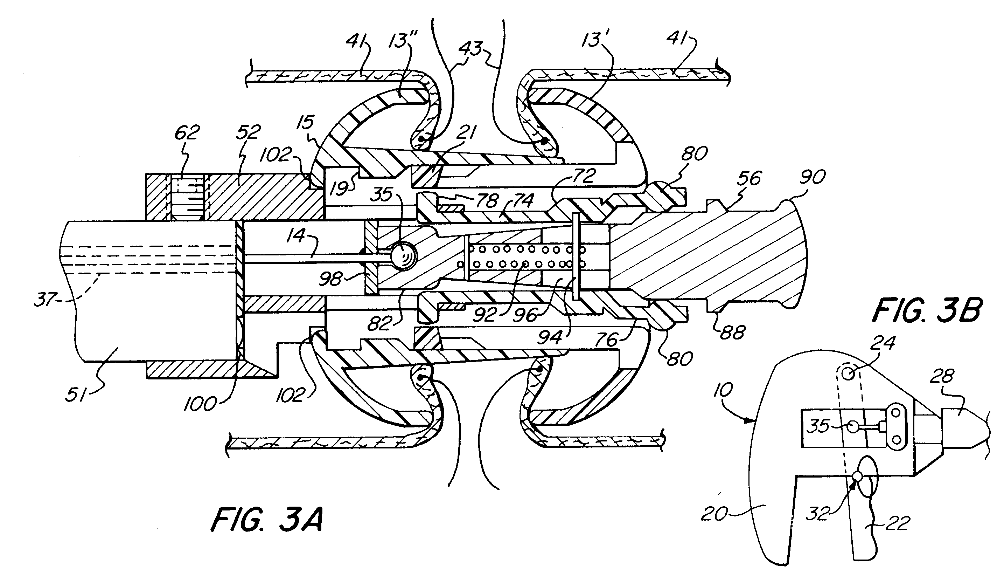

Before the endoscopic insertion device is inserted into the body, the anastomosis ring is mounted on the adapter in the engaged position as shown in Figure 1B. In this position, the head of the proximal unitary member 13'' rests against a shoulder 102 on the collar and is prohibited thereby from moving in a first direction toward the proximal end of the insertion device (see Figure 3). Likewise, the distal unitary member 13' is prohibited from moving in a second direction toward the distal end of the insertion device by engagement of the head with the enlarged contact points 80 on the containing fingers of the cage. The unitary members are also prevented from moving toward each other by the arrangement of the pawl engagement members 21, the sliding fingers 74 and the proximal end 82 of the spool. The engagement members must flex radially inwardly to slide over the pawls and close the anastomosis ring. However, the engagement members are prevented from flexing radially inwardly by the sliding fingers 74, which themselves cannot flex radially inwardly because they are opposed to the proximal end of the spool. Therefore, until the sliding fingers can flex radially inwardly, the engagement members cannot slide over the pawls and the anastomosis ring cannot be closed. The internal spring and transverse pin biases the cage in the distal direction to keep the lips 78 of the sliding fingers opposite to the engagement members and ensure that the anastomosis ring member remains open in the engagement position.

With the anastomosis ring secured on the adapter, the insertion device inserts the anastomosis ring into a natural body orifice and through a tubular body member. For purposes of this example, the insertion device is inserted through the mouth and into the esophagus. As the insertion device is being inserted, the trigger on the handle body is locked to prevent movement of the spool.

The anastomosis ring is positioned to approximately straddle the portion of the esophagus to be treated. The esophagus is severed and the diseased or cancerous portions of the esophagus are cut out and removed. The insertion device is adjusted, if necessary, to position the unitary members in the open ends 41 of the esophagus as shown in Figure 3A. A purse string suture 43 is applied to each open end in a known manner. The suture is tightened to snugly draw each open end around the neck of its respective unitary member. At this point, the unitary members are ready to be closed to form the anastomosis ring.

To close the unitary members, the locking pin is removed from the trigger and the trigger is squeezed as shown in Figure 4B to pull the control rod in the first, or proximal, direction. This action on the control rod retracts the spool into the collar as shown in Figure 4A. The spool at first slides relative to the cage and moves the proximal end past the sliding fingers. This positions the truncated cone section 84 of the spool opposite to the sliding fingers. As the spool continues to retract, the abutting annular ring 88 on the spool contacts and drives the containing fingers on the cage in the first direction. The enlarged contact points 80 on the containing fingers in turn force the distal unitary member toward the proximal unitary member. This motion is made possible because the sliding fingers can now flex radially inwardly toward the truncated cone and allow the pawl engaging members to slide over the pawls. As the engaging members slide over the pawls, the anastomosis ring is locked in the closed position. The closed anastomosis ring secures therebetween the open ends of the esophagus to grow and heal together.

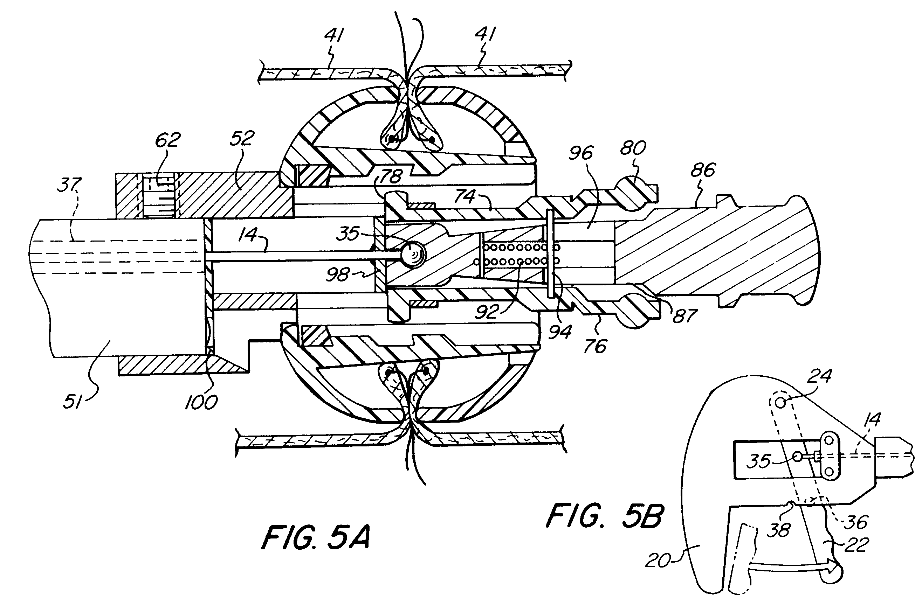

The trigger is then actuated in the opposite direction as shown in Figure 5B to release the anastomosis ring adapter from the anastomosis ring. This action pushes the control rod and spool in the second, or distal, direction as shown in Figure 5A. Initially, the spool slides axially in the distal direction relative to the cage. However, as the proximal end aligns itself with the sliding fingers, the outer periphery of the small washer 98 abuts the sliding fingers and moves the cage in the distal direction until the lips 78 on the sliding fingers engage the distal ends of the elongated slots 68. At this point, the cylindrical section 86 extends past the enlarged contact points on the containing fingers. As demonstrated in Figure 4A, the cylindrical section biases the containing fingers radially outwardly when it is in contact with the enlarged contact points. However, when the cylindrical section extends past the enlarged contact points as shown in Figure 5A, the containing fingers naturally flex radially inwardly toward a reduced diameter section 87 of the spool.

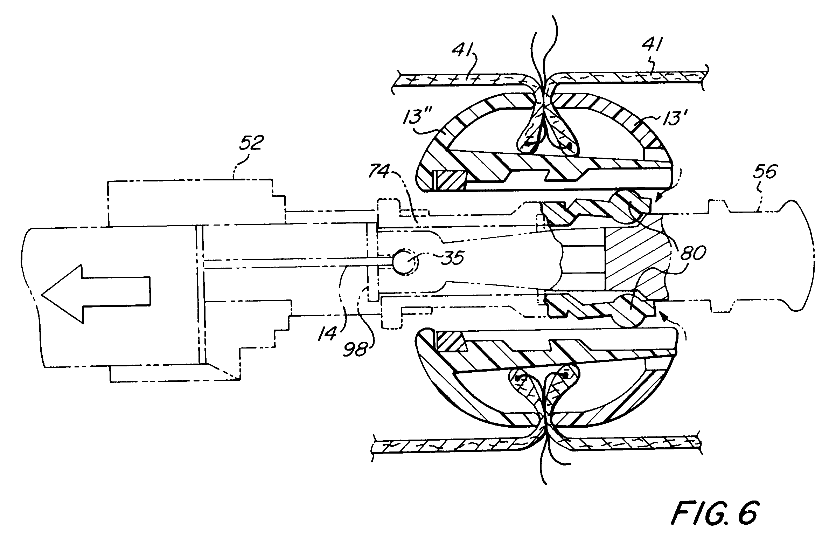

The entire insertion device can then be withdrawn through the body organ to release the adapter 16 from the anastomosis ring. As shown in Figure 6, the containing fingers are biased radially inwardly such that the enlarged contact points do not engage the head of the distal unitary member. The adapter can then be withdrawn axially through the anastomosis ring.

Although specific embodiments of the present invention have been described above in detail, it will be understood that this description is merely for purposes of illustration. Various modifications of and equivalent structures corresponding to the disclosed aspects of the preferred embodiments in addition to those described above may be made by those skilled in the art without departing from the spirit of the present invention which is defined in the following claims, the scope of which is to be accorded the broadest interpretation so as to encompass such modifications and equivalent structures.

## Claims

1. An endoscopic insertion device for inserting an anastomosis ring having two unitary members into an anatomic tubular body organ, comprising:
anastomosis ring adapter means for mounting two unitary members in an engaged position;
inserting means for inserting said adapter means in a tubular body organ; and
actuating means for actuating said adapter means to close the anastomosis ring and release said adapter means from the anastomosis ring.

2. An endoscopic insertion device according to Claim 1, said ring adapter means including slidable closing means for closing the anastomosis ring, and driving means for driving said closing means.

3. An endoscopic insertion device according to Claim 2, said ring adapter means further including a cylindrical collar for receiving said closing means and said driving means, with said closing means sliding in an axial direction relative to said cylindrical collar and said driving means sliding in an axial direction relative to said closing means.

4. An endoscopic insertion device according to Claim 3, said closing means including a plurality of sliding fingers for limiting axial movement of said closing means within said cylindrical member and containing fingers for abutting the anastomosis ring.

5. An endoscopic insertion device according to Claim 4, said driving means comprising a spool having a cylindrical section for biasing said containing fingers radially outwardly and a reduced diameter section for permitting said containing fingers to flex radially inwardly, wherein said containing fingers abut the anastomosis ring when they are biased radially outwardly by said cylindrical section.

6. An endoscopic insertion device according to Claim 5, said driving means further comprising a proximal end for preventing said sliding fingers from flexing radially inwardly and a truncated cone section for permitting said sliding fingers to flex radially inwardly.

7. An endoscopic insertion device according to Claim 6, further comprising first connecting means for connecting said adapter means to said inserting means.

8. An endoscopic insertion device according to Claim 7, further comprising second connecting means for connecting said adapter means to said actuating means.

9. An endoscopic insertion device according to Claim 8, said actuating means including a handle assembly with a pivotable trigger and a control cable connected at one end to said trigger and at a second end to said adapter means.

10. An endoscopic insertion device according to Claim 9, said actuating means further including a locking mechanism for locking said trigger in a locked position.

11. An endoscopic insertion device according to Claim 10, wherein said inserting means comprises an endoscope.
